# EUROPEAN PATENT APPLICATION

(11) **EP 4 385 477 A1**
(43) Date of publication of application: **19.06.2024**
(21) Application number: 22213633.5
(22) Date of filing: 14.12.2022
(51) Int. Cl.: A61F 13/49, B32B 3/08

(54) **ABSORBENT ARTICLE WITH IMPROVED FIT**

(71) Applicant: Ontex BV, 9255 Buggenhout (BE); Ontex Group NV, 9320 Erembodegem (BE)
(72) Inventor: BRAUN STREB, Hanne, 50389 Wesseling (DE); PANNWITT, Stefanie, 56727 Mayen (DE)
(74) Representative: Macchetta, Andrea

(57) **Abstract**

An absorbent article comprising: a chassis comprising a liquid permeable topsheet, a liquid impermeable backsheet, and an absorbent core positioned between said topsheet and backsheet, and comprising a longitudinal axis extending along a length of said chassis and a transverse axis being substantially perpendicular to the longitudinal axis and extending along a width of said chassis, wherein said length extends along the longest dimension of said chassis; one or more, preferably at least two, discrete elastically elongatable panels each comprising an imaginary panel axis dividing said panel(s) into two halves comprising an upper or back half and a lower or front half wherein the lower or front half is generally positioned closer to a front portion of the article compared to said upper or back half; and one or more fastening members joined to each of said one or more discrete elastically elongatable panels, and comprising an imaginary fastener axis; wherein the one or more fastening members are joined to the upper or back half of said discrete elastically elongatable panels, and wherein the panel axis and the fastener axis are offset, and wherein said panels comprise at least one cover layer and an elastomeric film attached to the cover layer.

## Description

### TECHNICAL FIELD

The disclosure relates to absorbent articles such as disposable absorbent articles, preferably selected from diapers (whether for baby or adults).

### BACKGROUND

Disposable absorbent articles, such as diapers, are designed to contain bodily wastes and prevent soiling of the wearer's clothing and/or other items (e.g., a bed, a chair, a blanket, etc.). The fit of the article to the wearer's body is important in ensuring that these wastes are contained. Such articles are also designed to be cost-effective, and therefore manufacturers generally make the articles applicable for use by individuals with a wide range of body types. Accordingly, new and improved disposable absorbent articles that both conform to a wide range of body types and fit snuggly to the user to contain wastes and limit leakage are of continued interest.

One way in which manufacturers attempt to balance the competing interests of proper fit and variation in body type is through the use of expandable materials. One such group of materials is known as stretch laminates. As the name suggests, these materials are actually composites of individual components that are laminated together, through the use of an adhesive, for example. A typical stretch laminate will attempt to combine one or more layers of cover material with one or more layers or strands of an elastomeric material.

Complications arise in that stretch laminates are notoriously difficult and expensive to manufacture. Considerable effort has gone into proposing new types of stretch laminates and new methods for the fabrication of stretch laminates.

For example, EP 3 213 728 A1 relates to transversely extensible elastic laminar web materials comprising - a first and a second web material each of which defines a first and a second distal region adjacent to corresponding longitudinal side edges and a central region between the aforesaid distal regions - at least one web of elastomeric material applied to these central regions of the first and second web materials and - a plurality of connection formations applied to at least one distal region of said first and second web materials, and projecting from a respective longitudinal edge. In the transversely extensible elastic laminar web material, the elastomeric web material and the connection formations are interposed between said first and second web materials and are joined thereto by mechanical welds.

EP 3 496 692 A1 relates to an absorbent article that includes a first waist region, a second waist region and a crotch region disposed between the first and second waist regions; and a chassis comprising a topsheet, a backsheet and an absorbent core disposed between the topsheet and the backsheet. The absorbent article also includes an ear disposed in one of the waist regions. The ear includes a laminate having a first nonwoven and a second nonwoven and an elastomeric material sandwiched between said first and second nonwovens in an elasticized region. The laminate also includes a first bonding region comprising a first plurality of ultrasonic bonds having a first bond density, and a second bonding region comprising a second plurality of ultrasonic bonds having a second bond density. The first bond density is greater than the second bond density.

Some of these drawbacks have been overcome as described for example in EP 4 052 688 A1 that relates to an absorbent article comprising: a chassis comprising a liquid permeable topsheet, a liquid impermeable backsheet, and an absorbent core positioned between said topsheet and backsheet, and comprising a longitudinal axis extending along a length of said chassis and a transverse axis being substantially perpendicular to the longitudinal axis and extending along a width of said chassis, wherein said length extends along the longest dimension of said chassis; and at least one elastically elongatable panel joined to the chassis, wherein the elastically elongatable panel comprises: at least one cover layer; and an elastomeric film attached to the cover layer, wherein said least one cover layer and elastomeric film are joined by mechanical bonding, at a plurality of discrete bonding elements and wherein each said panel comprises a plurality of wrinkles having peaks and troughs formed on at least one of said cover layer, wherein said panel comprises an average of no more than two, preferably no more than one, wrinkles between two consecutive discrete bonding elements along a panel length extending substantially parallel to the transverse axis, and wherein said panel comprises a wrinkle distribution of at least 1.1 wrinkles/mm.

There is however a continuing desire to provide products that maintain optimum fit but use the least amount of material hence providing further cost advantages.

### SUMMARY

The disclosure relates to an an absorbent article comprising: a chassis comprising a liquid permeable topsheet, a liquid impermeable backsheet, and an absorbent core positioned between said topsheet and backsheet, and comprising a longitudinal axis extending along a length of said chassis and a transverse axis being substantially perpendicular to the longitudinal axis and extending along a width of said chassis, wherein said length extends along the longest dimension of said chassis; one or more, preferably at least two, discrete elastically elongatable panels each comprising an imaginary panel axis dividing said panel(s) into two halves comprising an upper or back half and a lower or front half wherein the lower or front half is generally positioned closer to a front portion of the article compared to said upper or back half; and one or more fastening members joined to each of said one or more discrete elastically elongatable panels, and comprising an imaginary fastener axis; wherein the one or more fastening members are joined to the upper or back half of said discrete elastically elongatable panels, and wherein the panel axis and the fastener axis are offset, and wherein said panels comprise at least one cover layer and an elastomeric film attached to the cover layer.

### BRIEF DESCRIPTION OF THE FIGURES

**Fig. 1** Illustrates an absorbent article according to an embodiment of the present disclosure.
**Fig. 2A-B** Illustrates absorbent articles according to an embodiment of the present disclosure.
**Fig. 3** Illustrates a cross-section at a position A-A of Fig. 2A.
**Fig. 4** Illustrates an elastically elongatable panel according to an embodiment of the present disclosure.
**Fig. 5** Illustrates a schematic illustration of a bonding pattern for an elastically elongatable panel according to an embodiment of the present disclosure.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed disclosure. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation.

The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

"Laminate" refers to elements being attached together in a layered arrangement.

By "substantially", it is meant at least the majority of the structure referred to.

"Elastic," "elastomeric," and "elasticized/elasticised" herein may mean the ability of a material to stretch by at least 100% without rupture or breakage at a given load, and upon release of the load the elastic material or component exhibits at least 70% recovery (i.e., has less than 30% set) in one of the directions as per the Hysteresis Test described herein. Stretch, sometimes referred to as strain, percent strain, engineering strain, draw ratio, or elongation, along with recovery and set may each be determined according to the Hysteresis Test described in more detail below. Materials that are not elastic are referred as inelastic. "Extensible" typically means the ability to stretch or elongate, without rupture or breakage, by at least 50% as per step 5(a) in the Hysteresis Test herein (replacing the specified 100% strain with 50% strain).

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

The term "absorbent article" refers to a device that absorbs and contains liquid, and more specifically, refers to a device that is placed against or in proximity to the body of the wearer to absorb and contain the various wastes/exudates discharged from the body.

The terms "activated" and "pre-activated" refer to a process of mechanically deforming a material in order to increase the extensibility of at least a portion of the material. A material may be activated or pre-activated by, for example, incrementally stretching the material in at least one direction.

The terms "adhesively bonded" or "adhesively laminated" refer to a laminate wherein an adhesive is used to bond an elastomeric material to at least one cover layer.

The term "attached" refers to elements being connected or united by fastening, adhering, bonding, or by any other method suitable for connecting the elements together and to their constituent materials. Many suitable methods for attaching elements together are well-known, including adhesive bonding, pressure bonding, thermal bonding, ultrasonic bonding, mechanical fastening, etc. Such attachment methods may be used to attach elements together over a particular area either continuously or intermittently.

The term "diaper" refers to an absorbent article generally worn by infants or incontinent persons about the lower torso and having the general form of a sheet, different portions of which are fastened together to encircle the waist and the legs of the wearer.

The term "disposable" refers to absorbent articles that generally are not intended to be laundered or otherwise restored or reused as absorbent articles, i.e., they are intended to be discarded after a single use and, preferably, to be recycled, composted or otherwise disposed of in an environmentally compatible manner.

The term "disposed" is used to mean that an element(s) is formed (joined and positioned) in a particular place or position as a unitary structure with other elements or as a separate element joined to another element.

The term "extensible" refers to the property of a material, wherein: when a biasing force is applied to the material, the material can be extended to an elongated length of at least 110% of its original relaxed length (i.e., can extend 10%), without a rupture or breakage that renders the material unusable for its intended purpose. A material that does not meet this definition is considered inextensible. In some embodiments, an extensible material may be able to be extended to an elongated length of 125% or more of its original relaxed length without rupture or breakage that renders the material unusable for its intended purpose. An extensible material may or may not exhibit recovery after application of a biasing force. Throughout the present disclosure, an extensible material is considered to be "elastically extensible" if, when a biasing force is applied to the material, the material can be extended to an elongated length of at least 110% of its original relaxed length (i.e., can extend 10%), without rupture or breakage which renders the material unusable for its intended purpose, and when the force is removed from the material, the material recovers at least 40% of its elongation. In various examples, when the force is removed from an elastically extensible material, the material may recover at least 60%, or at least 80%, of its elongation.

The terms "interior" and "exterior" refer respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside", or "body-facing" and "garment-facing". Also, when the absorbent article is oriented such that its interior faces upward, e.g., when it is laid out in preparation for setting the wearer on top of it, synonyms include "upper" and "lower" and "top" and "bottom", respectively.

The term "joined" refers to configurations whereby an element is directly secured to another element by attaching the element directly to the other element, and configurations whereby an element is indirectly secured to another element by attaching the element to intermediate member(s) which in turn are attached to the other element.

The term "lateral" or "transverse" refers to a direction running at a 90 degree angle to the longitudinal direction and when combined with the term "substantially" includes directions within ±45° of the lateral direction.

The term "longitudinal" refers to a direction running parallel to the maximum linear dimension of the article and when combined with the term "substantially" includes directions within ±45° of the longitudinal direction.

"Breathable" as used herein is the ability of the structure referred to (e.g. a layer(s), laminate etc.) of allowing water vapor to permeate therethrough and typically that the structure referred to has a water vapor transmission rate (WVTR) of at least 1500 grams/m² - 24 hours, preferably at least 2500 grams/m² - 24 hours, according to the method described herein.

The term "recovery" refers to ability of a material to return to its original size after it has been stretched.

The term "refastenable" refers to the property of two elements being capable of releasable attachment, separation, and subsequent releasable reattachment without substantial permanent deformation or rupture.

The terms "releasably attached," "releasably engaged," and variations thereof refer to two elements being connected or connectable such that the elements tend to remain connected absent a separation force applied to one or both of the elements, and the elements being capable of separation without substantial permanent deformation or rupture. The required separation force is typically beyond that encountered while wearing the absorbent garment.

The term "wrinkle" as used herein refers to a fold, ridge or crease.

The term "film" refers to a sheet-like material wherein the length and width of the material far exceed the thickness of the material. Typically, films have a thickness of about 0.5 mm or less.

Embodiments of the articles and processes according to the disclosure will now be described. It is understood that technical features described in one or more embodiments maybe combined with one or more other embodiments without departing from the intention of the disclosure and without generalization therefrom, especially when such combinations are explicitly or implicitly inferred.

### THE ABSORBENT ARTICLE

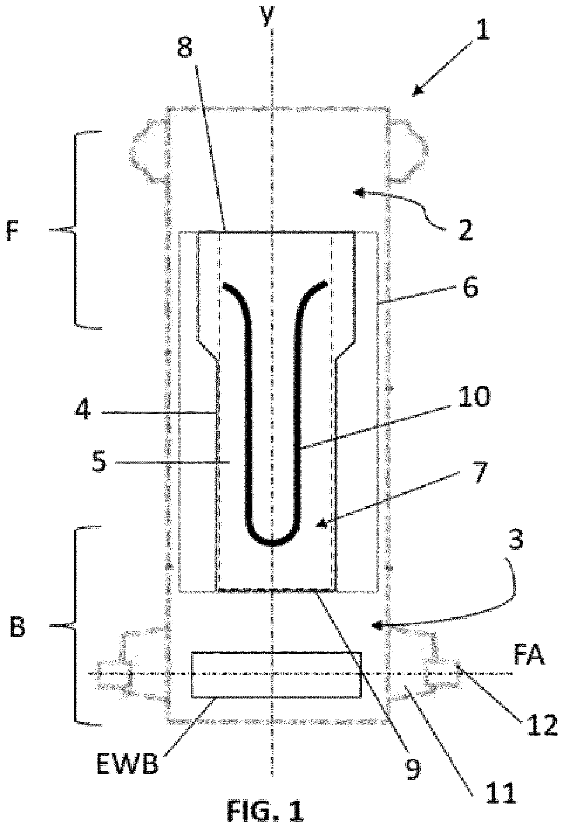

As exemplified in Fig. 1, absorbent articles herein comprise a chassis comprising a liquid permeable topsheet (2), a liquid impermeable backsheet (3), and an absorbent core (4) positioned between said topsheet (2) and backsheet (3), and comprising a longitudinal axis (y) extending along a length of said chassis and a transverse axis being substantially perpendicular to the longitudinal axis and extending along a width of said chassis, wherein said length extends along the longest dimension of said chassis. Articles herein are preferably selected from diapers.

The absorbent core (4) preferably comprises an absorbent material (5), said absorbent material comprising cellulose fibers and/or superabsorbent polymers, preferably wherein said absorbent material is contained within at least one core wrap substrate (6) enclosing said absorbent material therein, typically wherein the superabsorbent polymers are in the form of particles or granules, fibers, and mixtures thereof. The core wrap is typically a nonwoven selected from the group consisting of spunbond (S), spunbond-meltblown (SM), spunbond-metlblown-spunbond (SMS), spunbond-meltblown-metlblown-spunbond (SMMS), spunbond-spunbond-metlblown-spunbond (SSMS), spunbond-spunbond-metlblown-spunbond-spunbond (SSMSS), and combinations thereof. The core wrap may also, or alternatively, comprise a carded nonwoven preferably a carded thermobonded nonwoven wherein the thermobonds are formed by calendering (i.e. a carded nonwoven free of air-through bonding).

The absorbent article may further comprise an acquisition distribution layer (ADL) (7) positioned between the topsheet (2) and an upper layer of the core wrap (5). The ADL may be in good and/or direct contact with both the topsheet (2) and an upper layer of the core wrap (5) over the majority of its surface area. ADLs customary in the art may be used such as nonwovens selected from spunbond or carded thermobonded whether air-through bonded or calendered. Also ADLs comprising cellulosic fibers and/or polylactide are suitable.

The absorbent article typically comprises a front region (F), a back region (B), and a crotch region positioned between the front region (F) and back region (B) such that the longitudinal axis (y) crosses each of said front region (F), crotch region and back region (B) generally in this order. The crotch region of the article typically being positioned to fit between the wearer's legs when the article is worn by a subject.

The absorbent core (4) of the article (1) may comprise a front transversal edge (8) generally positioned at or proximal to the front region (F) and a back transversal edge (9) generally positioned at or proximal to the back region (B), and typically connected to each other by oppositely disposed lateral edges extending along the longitudinal axis (y). The transversal edges (8, 9) and lateral edges together forming the perimeter of said core (4)

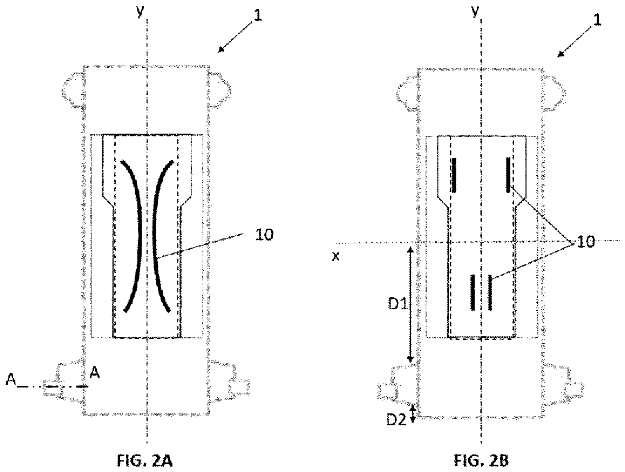

The absorbent core may further comprise one or more channels (10) substantially free of absorbent material, typically meaning less than 15%wt or less than 5% of absorbent material within said channels. Preferably, a top layer of the core wrap (5) is bonded to a bottom layer of the core wrap (5) in one or more attachment zones corresponding to said channels (10) thereby allowing the channels to be substantially free of absorbent material or even essentially entirely free of absorbent material. The bonding may be achieved via adhesive and/or mechanical bonding. Although Fig. 1 illustrates a single said channel (10) other channel shapes and/or plural channels are also contemplated as exemplified, without any limitation intended, in Figs. 2A-B.

The absorbent articles herein comprise at least one elastically elongatable panel (11) joined to the chassis as will be explained more in detail in the section that follows. Said panel(s) are typically laterally extending in the back portion (B) of the article (1) when the article is a diaper, whilst when the article is a pant may be rather front and/or back panels forming front and/or back belts at the front portions (F) and/or back portion (B).

The panels (11) herein are preferably discrete panels generally meaning that they are separate components that are individually joined to the chassis. Panels (11) herein are preferably symmetric meaning that each discrete panel taken in isolation comprises at least one axis of symmetry.

In an embodiment, panels (11) herein are substantially square or rectangular in shape. Advantageously an increased surface area is thus attained that wraps on the waist of the wearer compared to shaped elastic panels.

The elastically elongatable panel(s) (11) may each comprise a fastening member (12) arranged to fasten to a frontal area of the external surface of the article. Typically the fastening member (12) comprises adhesive and/or mechanical hooks that mate and/or couple to a nonwoven layer such as a landing zone positioned at the external surface of the article at a belly position when worn by a subject.

In an embodiment, the at least one elastically elongatable panel (11) is joined to the chassis by mechanical bonding or adhesive at a first attachment zone of said panel, and preferably wherein said panel (11) is joined, preferably by mechanical bonding or adhesive, to a fastening member (12) at a second attachment zone of said panel oppositely disposed from said first attachment zone at an opposite end of said panel, and preferably wherein said absorbent article (1) comprises at least two of said panels (11) extending outboard of the chassis and oppositely disposed about the longitudinal axis (y) such that a centerline of said at least two of said panels (11) substantially correspond. Such advantageously allows effective construction of refastenable diapers and/or pants.

The elastically elongatable panels (11) herein comprise an imaginary panel axis (PA) generally being the centerline of the said elongatable panels (11) and extending substantially perpendicular to the longitudinal axis (y) of the article (1). The panel axis (PA) generally dividing the panels (11) herein into two halves comprising an upper (or back) half and a lower (or front) half wherein the lower (or front) half is generally positioned closer to the front region (or portion) (F) of the article compared to said upper (or back) half.

The fastening members (12) herein comprise an imaginary fastener axis (FA) generally being the centerline of the said fastening members (12) and extending substantially perpendicular to the longitudinal axis (y) of the article (1). The fastener axis (FA) of at least two (preferably each) oppositely disposed fastening members (12) substantially coincide (meaning that the at least two (preferably each) oppositely disposed fastening members (12) share the same fastener axis) and crosses the longitudinal axis (y) when viewing the article in an open and laid flat position as exemplified in Fig. 1. Advantageously this allows for symmetry about the y-axis and uniform encircling of the waist upon extension of the panels.

In a preferred embodiment, the upper (or back) half of the panel(s) (11) herein has a first engineering strain and the lower (or front) half of the panel(s) (11) herein has a second engineering strain, wherein the first and second engineering strains are different, preferably wherein the first engineering strain is lower than the second engineering strain, preferably from 10% to 85% lower than the second engineering strain. Advantageously this allows to compensate nonwoven deformation when using an offset fastening.

Preferably, at least a portion of the fastening members (12) herein overlap the panel axis (PA) generally so that the panel axis (PA) crosses said portion of the fastening members. Advantageously this allows for a force vector to be applied also to the lower half of the panels when extending said panels by pulling on the fastening member.

Preferably, the upper half of the panel(s) (11) comprises a first bond area density and the lower half of the panel(s) (11) comprises a second bond area density, wherein the first bond area density is different from said second bond area density, preferably said first bond area density is greater than the second bond area density, preferably from 20% to 100% greater than the second bond area density. The bond area density may be measured by taking the sum of the individual bond areas divided by the total area of the panel, and may be determined by standard image analysis (e.g. ratio of pixels by converting an image into black&white since the bonds will appear as black and the non-bonds as white the ratio of black pixels over white pixels can be taken to determine the bond area density). Preferably the bonds are in the form of a plurality of discrete bonds that may be formed by mechanical bonding as described in embodiments herein below in more detail. Advantageously this arrangement allows to attain panels having different elasticity and/or engineering strains in the upper and lower halves of the panel.

The panel axis (PA) and the fastener axis (FA) are offset generally such that the panel axis (PA) and the fastener axis (FA) are separated from each other by an axial distance (d_{A}) generally along an axis substantially parallel to the longitudinal axis (y) and substantially perpendicular to the panel axis (PA) and/or the fastener axis (FA). Advantageously this allows for improved fit that accommodates a wider range of subject sizes without having to increase the chassis length and/or add more material.

The fastening members (12) are preferably positioned in the upper (or back) half of the panels (11) herein and generally adjacent a shortest length thereof. Advantageously this allows to increase the overall product length without increasing the length of the chassis and hence achieving a material saving.

Preferably, the fastening members (12) are discrete generally meaning that they are not formed together with the panels (11) herein but are rather separate components that are joined thereto. Typically the discrete fastening members (12) are in the form of substantially rectangularly-shaped tabs (such as tape-fasteners). Advantageously this allows for processing efficiencies when applying such fastening members (12) in an asymmetrical arrangement as described herein.

In an embodiment, the panels (11) herein comprise a longest length (Lmax_{P}) extending substantially parallel to the longitudinal axis (y) and generally positioned most adjacent to the chassis. The longest length may generally correspond to a panel joint length of a panel joint region, the panel joint region being that portion of the panel(s) (11) that is joined to the chassis. The panels (11) may comprise a shortest length being located opposite the longest length (Lmax_{P}) and generally positioned furthest away from the chassis.

The axial distance (d_{A}) is preferably from 0.03Lmax_{P} to 0.50Lmax_{P}, more preferably from 0.04 Lmaxp to 0.25Lmax_{P}, even more preferably from more than 0.04 Lmax_{P} to less than 0.20Lmax_{P}, even more preferably from 0.05 Lmax_{P} to 0.15Lmax_{P}. Advantageously, operating within these ranges allows for an optimal balance of product length increase and fit whilst minimizing risk of tearing.

The panels (11) herein are generally positioned at a panel distance (D1) away from an article transversal centerline (x) (typically running perpendicular to and crossing the longitudinal axis y) towards the back (B) of the article. The panel distance (D1) typically being the distance between said transversal centerline (x) and the closest portion of the panel (11) thereto. Preferably, the ratio of the panel longest length and panel distance (Lmax_{P}/ D1) is from 0.30 to 1.0, preferably from 0.35 to 0.9, even more preferably from 0.40 to 0.85, even more preferably from 0.45 to 0.8. The panels (11) are generally positioned such that they do not extend beyond a terminal transversal edge (TE) of the article and preferably wherein a second panel distance (D2), extending from the terminal transversal edge (TE) and the closest portion of the panel (11) thereto, is greater than zero (typically from 2mm to 20mm, preferably from 5mm to 15mm, even more preferably from 7mm to 12mm) Advantageously this benefits fit onto the wearer when an offset tape is particularly used.

As exemplified in Fig.1, articles herein preferably comprise an elastic waistband (EWB) generally positioned on the back portion (B) of the article (1). The elastic waistband (EWB) is typically positioned between the absorbent core and a terminal transversal edge (TE) of the article and spaced therefrom along the longitudinal axis (y). The fastener axis (FA) is preferably arranged to extend along the centerline of the fastening members (12) and cross the elastic waistband (EWB) and the longitudinal axis (y). The elastic waistband (EWB) is generally positioned to overlap the fastener axis (FA) and is positioned such that the longest length of the elastic waistband (EWB) is substantially parallel to an article transversal centerline (x). Advantageously, ensuring that the fasteners are in-line with the elastic waistband reduces risk of deformation (or formation of wrinkles) of portions of the chassis between the elastic waistband and the terminal transverse edge in extended state, that may otherwise impact fit. Moreover, the elastic waistband positioned in this manner synergistically co-operates with the offset fastening member positioning for linear extension of the elastic side panels when pulling onto the fastening members.

In a preferred embodiment the elastic waistband (EWB) has an aspect ratio (shortest length parallel to the longitudinal axis y divided by longest length parallel to the transversal axis x) of from 3.5 to 8.5, preferably from 4 to 8, more preferably from 4.5 to 7.5. Advantageously this allows for limiting use of film material and hence reducing cost.

Preferably, the shortest length of the elastic waistband (EWB) is from 15% to 50%, preferably from 20% to 45%, even more preferably from 25% to 40%, of the panel(s) (11) longest length (Lmax_{P}). Advantageously this allows for reduced cost whilst limiting compromises on fit.

In specific embodiments herein referring to the presence of an elastic waistband (EWB) it is to be understood that the elastic film (14) of panel(s) (11) described herein may be alternatively replaced with elastic strands (such as manufactured and sold by the Lycra Company under the brand Lycra^{®}) to achieve similar synergistic benefits described (e.g. in relation to the fastener axis crossing and/or overlapping the discrete elastic waistband).

Absorbent articles herein may further comprise additional components such as lateral and/or transversal cuffs arranged to provide liquid leakage barriers at the longitudinal and/or transversal edges of the article respectively; frontal (typically inelastic) panels; one or more skin care lotions on the topsheet; one or more odor control compositions and/or fragrances contained in one or more layers beneath the topsheet; and other components common in the art of diapers and pants.

### ELASTIC PANEL(S)

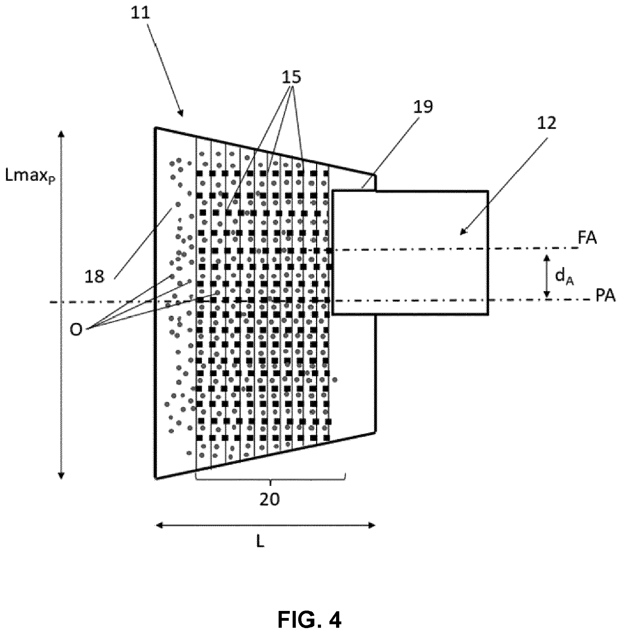

Elastically elongatable panel(s) (11) herein are joined to the chassis, and may comprise: at least one cover layer (13, 13'), preferably at least two cover layers; and an elastomeric film (14) attached to the cover layer(s) (13, 13') (in case of at least two cover layers, the film being sandwiched between at least two cover layers), wherein said least one cover layer (13, 13') and elastomeric film (14) are preferably joined by mechanical bonding at a plurality of discrete bonding elements (15), and preferably wherein each said panel (11) comprises a plurality of wrinkles having peaks (16) and troughs (17) formed on at least one of said cover layer (13, 13'), and wherein the said panel (11) may comprise a plurality of openings (O) extending through the film (14) and/or the cover layer(s) (13, 13') and wherein said openings (O) may be positioned outboard and/or inboard of said discrete bonding elements (15) so that said openings (O) and said bonding elements (15) do not substantially coincide. Partial overlap of said openings (O) and said bonding elements (15) may be acceptable but is less preferred, most preferably the openings and discrete bonding elements are positioned differently so as to not coincide and not overlap. It is to be noted that although the embodiment of Fig.4 is shown with respect to linearly patterned discrete bonding elements, the same may also apply (i.e. readily combinable) to sinusoidally patterned discrete bonding elements according to the other respective embodiments herein. Advantageously, this allows for added breathability as an elastic laminate free of adhesives (i.e. hydrophobic barriers) may be attained that further contains openings for added water vapor permeability whilst at the same time limiting the risk of tearing when the laminate is extended.

Elastically elongatable panel(s) (11) herein may be joined to the chassis, and comprise: at least one cover layer (13, 13'), preferably at least two cover layers; and an elastomeric film (14) attached to the cover layer(s) (13, 13') (in case of at least two cover layers, the film being sandwiched between at least two cover layers), wherein said least one cover layer (13, 13') and elastomeric film (14) are joined by mechanical bonding at a plurality of discrete bonding elements (15), and wherein each said panel (11) comprises a plurality of wrinkles having peaks (16) and troughs (17) formed on at least one of said cover layer (13, 13'), and wherein the said panel (11) comprises openings and/or apertures (herein also referred to as orifice(s)) and wherein said panel (11) is breathable and has a water vapor transmission rate (WVTR) of less than 2300 g/m²×24hr at an elongation of 0% and a water vapor transmission rate (WVTR) of more than 2800 g/m²×24hr at an elongation of 100%, as measured according to the test method herein. Advantageously, incrementally increasing breathability with % elongation allows for improved breathability when needed most e.g. inuse and moreover it may be beneficial to have a higher breathability when the elastic panels are extended to 100% or more elongation since the total surface area of such panels coming in contact with the wearer's skin is generally increased and hence capable of providing an exponential benefit in terms of comfort and skin well-being of the wearer. Typically the water vapor transmission rate (WVTR) values herein are average values as described in the method herein.

Preferably, the panel(s) (11) are selectively breathable in that they are non-breathable at an elongation of 20% or less and are breathable at an elongation of more than 40%, preferably from 60% to 140%. Advantageously this allows for an activatable breathability that becomes available to the wearer when stretching the panels for fitting. The panel(s) may comprise indicia on at least one of the film or cover layer(s) that become visible once the panel(s) becomes breathable. The indicia may be a printed image or color. For example, an image may be printed on an inner surface (facing the film) of the cover layer(s) prior to lamination to the film as described in processes herein, this resulting in the image not being visible (or only partially visible) at an elongation of 20% when the cover layer(s) is contracted to form gathers/wrinkles as described herein, and once extended to elongations of greater than 40% the said image becomes recognizable (or fully visible) by a user.

Alternatively or in addition to the above, the elastically elongatable panel(s) (11) herein may be joined to the chassis, and comprise: at least one cover layer (13, 13'), preferably at least two cover layers; and an elastomeric film (14) attached to the cover layer(s) (13, 13') (in case of at least two cover layers, the film being sandwiched between at least two cover layers), wherein said least one cover layer (13, 13') and elastomeric film (14) are joined by mechanical bonding at a plurality of discrete bonding elements (15), and wherein each said panel (11) comprises a plurality of wrinkles having peaks (16) and troughs (17) formed on at least one of said cover layer (13, 13'), and wherein the said panel (11) comprises openings and/or apertures (herein also referred to as orifice(s)) and wherein said panel (11) is breathable and has a water vapor transmission rate (WVTR) of more than 3500 g/m²×24hr, preferably from 4000 g/m²×24hr to 6000 g/m²×24hr, at an elongation of 140%, as measured according to the test method herein and wherein the water vapor transmission rate (WVTR) ratio at an elongation of 140% to 40% is greater than 1.35. Advantageously this arrangement allows for improved breathability when needed most.

In an embodiment, the openings (O) have an average diameter of from 50µm to 950µm, preferably from 80µm to 900µm, even more preferably from greater than 100µm to 850µm, even more preferably from 150µm to 750µm, even more preferably from 200µm to 650µm, even more preferably from 250µm to 550µm; and/or wherein the orifices (Or) have an average diameter that is less than the average diameter of the openings (O). Average diameter may be determined by standard image analysis via a microscope as known in the art and over one or more areas that may correspond to squares of 1cm × 1cm (or more) at one or more locations (that may be random and different) of the laminate. Advantageously, such sizing aids to provide enhanced breathability whilst limiting risk of tearing and/or mechanical strength of the laminate under load.

In an embodiment, the area occupied by the openings (O) and/or orifices (Or) is not more than 40% of the total surface area of the film (14), preferably not more than 35%, even more preferably from 5% to 30%, even more preferably from 10% to 25%. Advantageously this aids promotion of breathability whilst retaining structural integrity of the laminate.

In a preferred embodiment, the panel(s) (11) have a first bonding region having a first bond density (i.e. number of bonds/mm² according to the test method herein) and a second bonding region having a second bond density wherein the second bonding region is interposed between two oppositely disposed first bonding regions wherein the second bond density is greater than the first bond density, preferably wherein the second bond density is at least 6% greater than the first bond density (preferably from 10% to 350% greater, even more preferably from 20% to 300% greater, even more preferably from 25% to 310% greater, even more preferably from 50% to 250% greater, even more preferably from 80% to 200% greater), typically wherein the first/second bonding regions comprise discrete bonding elements (15) as described herein. The first bonding region preferably corresponds to a non-elasticised region of the panel(s) (11) and the second bonding region to an elasticised region of said panel(s) (11) generally interposed between oppositely disposed non-elasticised regions. Advantageously, this allows for increased wrinkle formation in the elastic region of the panel for good softness, and further improved breathability in the section of the panel where it is most needed particularly when orifices are formed in bonding elements as described herein.

In an embodiment, the openings (O) and the discrete bonding elements (15) have a pattern that is different, and wherein said pattern differs by average spacing, pattern uniformity, size, shape, orientation, aggregate area, aggregate pattern shape and combinations thereof. For example, smaller and more rounded shapes (e.g. circles or ellipses) may be beneficial for creating openings for breathability but may be less desirable for the formation of bonding elements where larger and polygonal (e.g. squares, rectangles etc.) may be more desirable to achieve increased surface area of bonding for added mechanical strength of the laminate.

Preferably, the openings (O) have an average surface area that is smaller than the average surface area of the discrete bonding elements (15), preferably at least 5% smaller, more preferably from 10% to 70% smaller. Advantageously this allows for enhanced breathability whilst limiting crack formation and/or tearing. Such area measurements are typically carried out on laminates in a relaxed state and generally using the following procedure: bond dimensions are measured to accuracy of 0.01 mm using a microscope and/or imaging software. At least 5 bonds taken at random from the same specimen/panel are measured. The dimensions for each bond are used to calculate the bond area as per the mathematical area formula for the given shape of the bond. The area of partial bonds inside the specimen may also be measured. An average surface area is calculated for the at least 5 bond measurements taken as described above per panel/specimen. A total of five specimen/panels are used and an average bond surface area is then calculated. The same method may be repeated for measuring the average surface area of the openings.

Preferably, the openings (O) have an aggregate area that is greater than the aggregate area of the the discrete bonding elements (15), preferably at least 5% greater, more preferably from 10% to 50% greater, even more preferably from 15% to 45% greater. Advantageously this allows for enhanced breathability whilst maximizing tear resistance. Such area measurements are typically carried out on laminates in a relaxed state and generally using the following procedure: aggregate area is calculated by summing the bond areas for each bond in the specimen. Bond dimensions are measured to accuracy of 0.01 mm using a microscope and/or imaging software. The dimensions for each bond are used to calculate the bond area as per the mathematical area formula for the given shape of the bond. The area of partial bonds inside the specimen are also measured. All bond areas within the specimen are added to calculate aggregate bond area for the specimen. A total of five specimen are used and an average is calculated. The same method is repeated for measuring the aggregate area of the openings.

In an embodiment, the plurality of discrete bonding elements (15) comprise an orifice (Or) forming an island within said bonding elements (15), and wherein said orifice has an elongate shape, typically measured with the panel (11) in relaxed state, preferably wherein the openings (O) have a shape that is different to that of said orifice. More preferably, the longest dimension of the elongate shape is substantially perpendicular to a stretch direction of the panel(s) (11) and generally corresponding to an axis that is substantially parallel to the longitudinal y axis of the article. Advantageously this allows to provide extra breathability whilst adding some resistance to crack propagation and tearing. Preferably, the openings (O) are made by laser or mechanical piercing such as by one or more needles or pins, preferably heated needles or pins; and the orifices are made by melt-fusion such as ultrasonic bonding, thermal pressure bonding and combinations thereof, preferably ultrasonic bonding.

In a preferred embodiment, the panel (11) is breathable and has a water vapor transmission rate (WVTR) of less than 2250 g/m2x24hr, preferably less than 2200 g/m2x24hr, preferably less than 2150 g/m²×24hr, preferably less than 2100 g/m²×24hr, even more preferably from 500 g/m²×24hr to 2000 g/m²×24hr, at an elongation of 0%; and a water vapor transmission rate (WVTR) of more than 2850 g/m²×24hr, preferably more than 2950 g/m²×24hr, more preferably more than 3000 g/m²×24hr, even more preferably from 3100 g/m²×24hr to 5500 g/m²×24hr, at an elongation of 100%, as measured according to the test method herein. Advantageously, a balance between excellent breathability and mechanical stability may thus be attained.

Preferably, the water vapor transmission rate (WVTR) ratio at an elongation of about 120% to 40% is greater than about 1.2, preferably from about 1.25 to 2.5; and/or wherein the water vapor transmission rate (WVTR) ratio at an elongation of about 140% to 40% is greater than about 1.35, preferably from about 1.4 to 3.5, even more preferably from about 1.5 to 3, even more preferably from 1.6 to 2.5, as measured according to the method described herein. Such ratios may be determined by following the water vapor transmission rate (WVTR) test method described herein at elongations of 40% and 140% respectively and then dividing the WVTR value measured at an elongation of 140% by the WVTR value measured at an elongation of 40%. Advantageously, such ratios aid to attain the correct increased permeability when most needed whilst reduce risk of weakened laminates (more prone to tearing) that may result in order to achieve higher breathability at lower % elongations.

In an embodiment, elastically elongatable panel(s) (11) herein are joined to the chassis, and comprise: at least one, preferably at least two, cover layers (13, 13'); and an elastomeric film (14) attached to the cover layer, wherein said least one cover layer (13, 13') and elastomeric film (14) are joined by mechanical bonding, at a plurality of discrete bonding elements (15), preferably having a shape selected from substantially square, rectangular, circular and combinations thereof, and wherein each said panel (11) comprises a plurality of wrinkles having peaks (16) and troughs (17) formed on at least one, preferably both, of said cover layer (13, 13'), wherein said panel (11) comprises an average of no more than two, preferably no more than one, wrinkles between two consecutive discrete bonding elements (15) along a panel length (L) extending substantially parallel to the transverse axis, and wherein said panel (11) comprises a wrinkle distribution of at least 1.1, preferably from 1.2 to 2.5, even more preferably from 1.3 to 2, wrinkles/mm, according to the method herein. Advantageously, a large number of wrinkles within the above ranges generally increases bulkiness and therefore actual and perceived softness of the material which is ever more important in elastic side panels which are in tight contact with the skin of the user, nevertheless if the number of wrinkles is too high bulkiness turns into roughness as the peaks tend to form sharper apexes at extremely short amplitudes therefore it is beneficial to even more preferably remain within the above cited preferred range.

In an embodiment, the film (14) is colored with a first color and wherein at least one, preferably both, of the cover layer (13, 13') is colored with a second color, preferably wherein the first color and the second color has a CIELab color space difference (ΔE) of about 16 or less, preferably from 3 to 15, even more preferably from 5 to 12, even more preferably from 6 to 10, wherein the color difference for a first color (L1, a1, b1) and a second color (L2, a2, b2), is calculated according to the following formula: ΔE =√(ΔL2 +Δa2 +Δb2) wherein: ΔL =L1-L2; Δa =a1 -a2; and Δb =b1 -b2. Advantageously, when combined with the wrinkles described herein, this allows to provide a further perception of depth and texture that enhances the overall perception of softness of the material. Color measurements are typically performed using a commercial flat bed scanner capable of 4800 dpi, at 16 bit color depth, such as an Epson Perfection V500 Photo scanner (Epson America, Long Beach, Calif.). Each scan is calibrated against Pantone standards, and measurements made using Adobe Photoshop CS3 Extended Edition (Adobe Systems, Inc, San Jose, Calif.). The sample is measured on the printed side of the substrate. For example, if a laminate consist of a colored nonwoven and a colored film where the film is sandwiched between nonwovens, the nonwoven(s) is removed before the color on the film is measured and then the removed nonwoven is separately measured. Scans are typically calibrated using the Pantone Process Colors standard from the Pantone Formula Guide-Uncoated Papers (Pantone, Carlstadt, N.J.). CIE L*a*b* values are measured for the Pantone standard for each color, i.e., Process Yellow U, Process Magenta U, Process Cyan U, Process Black U, and the White uncoated paper. Tristimulus colors are measured according to ASTM Method E1164-07 (Standard Practice for Obtaining Spectrophotometric Data for Object-Color Evaluation) using a Hunter Labscan XE (HunterLab, Reston, Va.) with HunterLab Universal Software vs. 4.10 with the following settings: Scale CIELAB, 0/45 StdMode, Area View 0.50 in., Port Size 0.70 in., UV filter Nominal During measurement the standard is backed using the white calibration plate provided by HunterLab. To increase the reliability of the measurement, each color should be measured at least in triplicate and averaged. The sample is placed on the scanner with the printed-side toward the sensor. The Pantone standard is also placed on the scanner such that the sample and standard are both captured in the same image. The scan is typically collected at 1200 dpi at 8 bit color depth into Photoshop for objects with a primary dimension of greater than 3 mm, and at 2400 dpi, 8 bit color depth for objects with a primary dimension of less than 3 mm. Within Photoshop, the image is transformed into a Lab, 8 bit image (note in this version of Photoshop, L*a*b* is imprecisely denoted as Lab). Using the "Levels" command, the L channel of the image is adjusted to read within 2 units for each of the yellow, magenta, cyan, black and white colors on the Pantone standard. L*a*b* values are measured using the Color Sampler Tool using an 11 by 11 average sample size.

In an embodiment, the elastomeric film comprises two surfaces and a skin on at least one of the surfaces. Preferably, the skin comprises a polyolefin selected from the group consisting of: metallocene polyethylene, low density polyethylene, high density polyethylene, linear low density polyethylene, very low density polyethylene, a polypropylene homopolymer, a plastic random poly(propylene/olefin) copolymer, syndiotactic polypropylene, metallocene polypropylene, polybutene, an impact copolymer, a polyolefin wax, and mixtures thereof.

The elastomeric film may be pre-activated before attachment to at least one cover layer. Advantageously this allows for increased extension/retraction performance of the laminate in a cost-effective manner.

Preferably, the elastomeric film comprises a polyolefin elastomer, preferably selected from the group consisting of: metallocene polyethylene, low density polyethylene, high density polyethylene, linear low density polyethylene, very low density polyethylene, a polypropylene homopolymer, a plastic random poly(propylene/olefin) copolymer, syndiotactic polypropylene, metallocene polypropylene, polybutene, an impact copolymer, a polyolefin wax, and mixtures thereof.

Typically the elastomeric film is a blown film such as made by use of a blown film extrusion process.

The elastomeric film may have a thickness of from about 15 µm to about 60 µm, preferably from about 20 µm to about 50 µm, preferably from about 30 µm and about 45 µm. The film may comprise an elastomeric polyolefin, and in some embodiments, a polyolefin blown film. Non-limiting examples of useful elastically extensible materials include propylene based homopolymers or co-polymers, or ethylene based homopolymers or co-polymers selected from the group consisting of: an elastic random poly(propylene/olefin) copolymer, an isotactic polypropylene containing stereoerrors, an isotactic/atactic polypropylene block copolymer, an isotactic polypropylene/random poly(propylene/olefin) copolymer block copolymer, a stereoblock elastomeric polypropylene, a syndiotactic polypropylene block poly(ethylene-co-propylene) block syndiotactic polypropylene tri-block copolymer, an isotactic polypropylene block region-irregular polypropylene block isotactic polypropylene tri-block copolymer, a polyethylene random (ethylene/olefin) copolymer block copolymer, a reactor blend polypropylene, a very low density polypropylene, a metallocene polypropylene, metallocene polyethylene, and combinations thereof. Additional non-limiting examples of useful elastically extensible materials include styrene-isoprene-styrene block copolymers, styrene-butadiene-styrene block copolymers, styrene-ethylene-butylene-styrene block copolymers, polyurethanes, ethylene copolymers, polyether block amides, and combinations thereof.

Preferably, the mechanical bonding as referred to in embodiments herein is selected from the group consisting of thermo-sealing, pressure-sealing, ultrasonic bonding, and combinations thereof, preferably ultrasonic bonding.

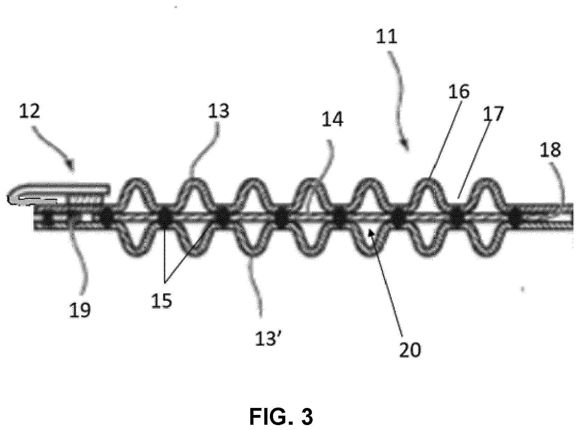

In an embodiment such as illustrated in Fig. 3, the at least one elastically elongatable panel (11) comprises a first and second inelastic region (18, 19) and an elasticized region (20) at least partially disposed between the first and second inelastic regions, and wherein the wrinkles are comprised in said elasticized region (20) and preferably wherein the first and second inelastic regions are free of said wrinkles, preferably wherein the first and second inelastic regions at least partly correspond to the first and second attachment zones respectively.

In a highly preferred embodiment, the plurality of discrete bonding elements (15) are disposed in a repeated pattern, preferably located within the elasticized region (20), and wherein said repeated pattern consists of a plurality of sinusoidally oriented discrete bonding elements (15) typically such that a plurality of sinusoidally shaped lines can be traced by connecting said discrete bonding elements (15) with an imaginary line along the panel length (L). Advantageously this allows for a multi-axis bulk-enhancing profile that permits to increase the softness of the laminate without necessarily increasing the basis weight of the cover layers. Moreover, the sinusoidal pattern provides for an increased visual perception of depth that enhances the softness perception of a user. Moreover, such pattern allows to enhance the accordion-like extension and retraction of the laminate.

Preferably, the sinusoidally oriented discrete bonding elements (15) are disposed substantially parallel to each other and each extend along the panel length (L), preferably wherein said sinusoidally oriented discrete bonding elements (15) are spaced apart from each other along an axis substantially perpendicular to said panel length (L).

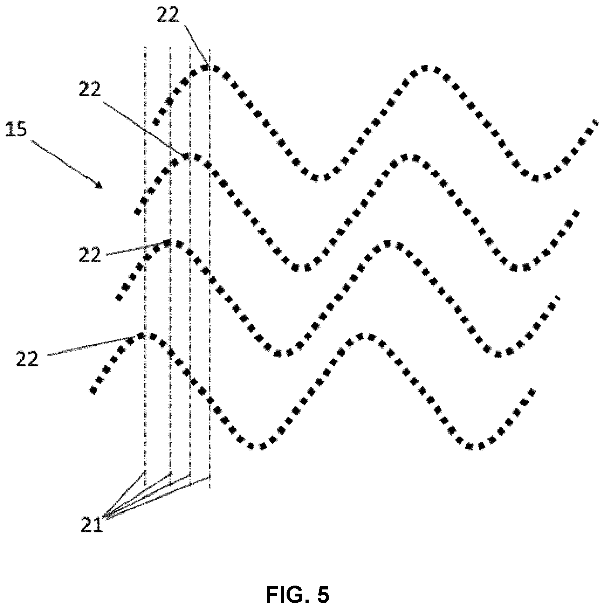

Preferably, as exemplified in Fig. 5, the sinusoidally oriented discrete bonding elements (15) are at least partly offset from each other such that when an imaginary crossing-line (21) is traced substantially perpendicular to the panel length (L) to cross at least one, preferably each, apex (22) of each of at least two, preferably at least three, even more preferably at least four, neighboring sinusoidally oriented discrete bonding elements (15); each said crossing-line is spaced apart from each other along an axis substantially parallel to the panel length (L). Advantageously this arrangement allows for further improved softness and perceived softness by adding a further dimension to the wrinkle formation.

Preferably, a first distance between neighboring discrete bonding elements (15) along an axis substantially parallel to the panel length (L) is less than a second distance between neighboring discrete bonding elements (15) along an axis substantially perpendicular to the panel length (L), preferably wherein the ratio of said first and second distances is from 0.10 to 0.80, preferably from 0.15 to 0.70, even more preferably from 0.18 to 0.60, even more preferably from 0.19 to 0.50, even more preferably from 0.20 to 0.45. Advantageously, this arrangement allows for the formation of the desired large amount of wrinkles per unit length whilst at the same time limiting the drawbacks of increased roughness as described above, especially when combined with the above described embodiments comprising a sinusoidal pattern.

In an embodiment the, preferably each, at least one cover layer (13, 13') is a nonwoven preferably having a basis weight of no more than 55 g/m² and preferably more than 17 g/m², preferably from 18 g/m² to 40 g/m², even more preferably from greater than 22 g/m² to less than 35 g/m², even more preferably from 23 g/m² to 30 g/m², as may be measured according to ASTM-D3776-9 method C (or other known standard methods in the art), and preferably wherein elastically elongatable panel comprises at least two cover layers (13, 13') positioned on opposite faces of the film (14) such that said film (14) is sandwiched between said cover layers and wherein the basis weight of said cover layers is different by a factor of at least 1.2, preferably at least 1.4, even more preferably from 1.5 to 3 (advantages being as described herein below in the process section relating to increased softness especially when openings are formed by mechanical piercing as described herein).. Preferably, wherein the nonwoven is selected from the group consisting of S, SM, SMS, SMMS, SSMS, and SSMSS, and preferably wherein said nonwoven comprises multi-component fibers typically comprising at least two, preferably at least three of: polyethylene, polypropylene, polyethylene terephthalate, copolyester of polyethylene terephthalate, acrylonitrile butadiene styrene, polylactide, and mixtures thereof. Advantageously this allows for added strength as well as softness of the laminate.

A suitable process that can be used for making elastically elongatable panels herein is described on paragraphs 0063 to 0095 of EP 3 213 728 A1 which is herein incorporated by reference. The salient modifications required in the taught process include the bonding pattern as described herein and wrinkle formation design.

A suitable activation device that may be employed is described on paragraphs 0041 to 0054 of EP 1 982 823 B1 which is herein incorporated by reference.

### Wrinkle distribution determination

The wrinkle distribution herein is determined by the following method.

For each side panel to be tested, the number of wrinkles is counted over a length of 10mm along the panel length (L) with the laminate in relaxed state (i.e. without applying an extension force between ends thereof, preferably taken at its "unused" state wherein "unused" herein means without the panel ever having been previously stretched since manufacture). The total number of wrinkles is then divided by 10 in order to provide the number of wrinkles per unit length. A total of 4 random locations within the elastic region (20) of the laminate may be measured accordingly and an average is calculated for each sample.

The 10mm length is typically taken from a starting position that encompasses at least one full wrinkle (i.e. a complete wrinkle comprising a peak positioned between two consecutive troughs) and by doing so if at the opposite extremity of the 10mm length only a partial wrinkle (i.e. not a complete wrinkle comprising a peak positioned between two consecutive troughs, e.g. the 10mm end at a position corresponding to the peak of a wrinkle) is formed, this is not counted as a wrinkle within the present method.

The above procedure is typically repeated for at least 4 samples of side panels and an average is calculated to provide the wrinkle distribution in wrinkles/mm as referred to herein.

As an example, the schematic of Fig. 6 illustrates 20 wrinkles over a length of 10mm along the panel length (L) with the laminate in relaxed state. If the same is observed at the 4 locations within the same sample and after examining a total of at least 4 samples, the wrinkle distribution in this example is 2 wrinkles/mm. Of course, it is understood herein that in case of variations/differences in number of wrinkles observed at the respective locations and/or samples, the wrinkle distribution is taken as the average as explained above.

### Bond density determination

The bond density herein is determined by the following method.

For each side panel to be tested, the number of bonds is counted over an area of 10mm x 10mm with the laminate in relaxed state (i.e. without applying an extension force between ends thereof, preferably taken in its "unused" state wherein "unused" herein means without the panel ever having been previously stretched since manufacture). The total number of bonds is then divided by 10 in order to provide the number of bonds per unit area (i.e. bonds/mm²). Bonds that overlap or partially overlap the perimeter of the 10x10mm area are included in the addition. At least 2 random locations within the elasticized region(s) of the laminate may be measured accordingly and an average is calculated, and the same is repeated for the non-elasticized region(s).

As an example, the image of Fig. 12 illustrates 10x10mm areas in the elasticized (A) and non-elasticized (B) regions respectively. The bond density in this example is about 3.7 bonds/mm in the elasticized region and about 1.2 bonds/mm in the non-elasticized region, with the bond density in the elasticized region being just under 310% greater than in the non-elasticized region.

### Hysteresis Test Method

The Hysteresis Test can be used to various specified strain values. The Hysteresis Test utilizes a commercial tensile tester (e.g., from Instron Engineering Corp. (Canton, MA), SINTECH-MTS Systems Corporation (Eden Prairie, MN) or equivalent) interfaced with a computer. The computer isused to control the test speed and other test parameters and for collecting, calculating, and reporting the data. The tests are performed under laboratory conditions of 23 °C + 2°C and relative humidity of 50% + 2%. The specimens are conditioned for 24 hours prior to testing.

The specimen is cut with a dimension of 10 mm in the intended stretch direction of the ear X 25.4 mm in the direction perpendicular to the intended stretch direction of the ear. A specimen is collected from either an inelastic region or from an elastic region.
1. Select the appropriate grips and load cell. The grips should have flat surfaces and should be wide enough to grasp the specimen along its full width. Also, the grips should provide adequate force and suitable surface to ensure that the specimen does not slip during testing. The load cell is selected so that the tensile response from the specimen tested is between 25% and 75% of the capacity of the load cell used.
2. Calibrate the tester according to the manufacturer's instructions.
3. Set the distance between the grips (gauge length) at 7 mm.
4. Place the specimen in the flat surfaces of the grips such that the uniform width lies along a direction perpendicular to the gauge length direction. Secure the specimen in the upper grip, let the specimen hang slack, then close the lower grip. Set the slack preload at 5 gram/force. This means that the data collection starts when the slack is removed (at a constant crosshead speed of 13mm/min) with a force of 5 gram force. Strain is calculated based on the adjusted gauge length (lini), which is the length of the specimen in between the grips of the tensile tester at a force of 5 gram force. This adjusted gauge length is taken as the initial specimen length, and it corresponds to a strain of 0%. Percent strain at any point in the test is defined as the change in length relative to the adjusted gauge length, divided by the adjusted gauge length, multiplied by 100.
5(a) First cycle loading: Pull the specimen to the 100% strain at a constant cross head speed of 70 mm/min. Report the stretched specimen length between the grips as Imax.
5(b) First cycle unloading: Hold the specimen at the 100% strain for 30 seconds and then return the crosshead to its starting position (0% strain or initial sample length, lini) at a constant cross head speed of 70 mm/min. Hold the specimen in the unstrained state for 1 minute.
5(c) Second cycle loading: Pull the specimen to the 100% strain at a constant cross head speed of 70 mm/min.5(d) Second cycle unload: Next, hold the specimen at the 100% strain for 30 seconds and then return the crosshead to its starting position (i.e. 0% strain) at a constant cross head speed of 70 mm/min.

A computer data system records the force exerted on the sample during the test as a function of applied strain. From the resulting data generated, the following quantities are reported:
i. Length of specimen between the grips at a slack preload of 5 gram-force (Imi) to the nearest 0.001 mm.
ii. Length of specimen between the grips on first cycle at the 100% strain (Imax) to the nearest 0.001 mm.
iii. Length of specimen between the grips at a second cycle load force of 7 gram-force (lext) to the nearest 0.001 mm.
iv. % Set, which is defined as (lext - Imi) / (Imax - Imi) * 100% to the nearest 0.01%.

The testing is repeated for six separate samples and the average and standard deviation reported.

### Breathability and WVTR test (or Moisture Vapor Permeability) method:

The moisture vapour (or vapor) permeability of a film or layer, or of a composite laminated structure comprising said film or layer, is measured via the Water Vapour Transmission Rate (WVTR) at 30°C and 15% relative humidity according to the ISO 15106-1:2003 test method. A Lyssy L80-5000 Water Vapor Permeation Analyser (manufactured and sold by Systech Instruments Ltd and Illinois Instruments Inc.) may be used.

The following modifications to the standard method are made: The test samples are clamped to a tensile testing machine (such as an Instron 5543 or similar) and stretched at a cross-head speed of 300mm/min at the desired elongation (such as 0%; 20%; 40%; 60%; 80%; 100%; 120%; and 140% elongations). As an example, a test sample having a length of 40mm at 0% elongation is stretched to 48mm length at 20% elongation; 56mm length at 40% elongation; 64mm length at 60% elongation; 72mm length at 80% elongation; 80mm length at 100% elongation; 88mm length at 120% elongation; 96mm length at 140% elongation; and so on.

For each elongation, the stretched test sample is then affixed to a self-adhesive sample card with an open area of 2.5cm² which is to be covered by the specimen that is subsequently tested according to the WVTR method described in the above referenced standard.

The sample card with stretched film sample is inserted into the test chamber of the Water Vapor Permeation Analyser. The lower test chamber has a saturated atmosphere maintained by a small water reservoir, while the upper chamber contains a sensitive, fastresponding relative humidity sensor. The upper chamber is first dried to a defined humidity level using dry air.

When the drying is complete, the airflow stops and the valves close. From that point, the chamber is a closed system, in which transmission of water vapour through the samples causes an increase in relative humidity in the upper chamber, which is recorded by humidity sensor in the chamber. The Water Vapor Permeation Analyser measures the time required for the upper chamber humidity to increase from a pre-defined lower limit to a pre-defined upper limit. The measured time interval is compared to the time obtained during calibration with a standard film of known permeability (Goretex^{®}) and the results is expressed as the water vapour transmission rate in g/m2/24h.

This test is repeated for different samples at each of the % elongations and results recorded. At least two WVTR (g/m2/24h) measurements are taken for each % elongation and an average WVTR is determined at each such elongation.

It is supposed that the present invention is not restricted to any form of realization described previously and that some modifications can be added to the presented embodiments without reappraisal of the appended claims.

## Claims

1. An absorbent article (1) comprising:
a chassis comprising a liquid permeable topsheet (2), a liquid impermeable backsheet (3), and an absorbent core (4) positioned between said topsheet (2) and backsheet (3), and comprising a longitudinal axis (y) extending along a length of said chassis and a transverse axis being substantially perpendicular to the longitudinal axis and extending along a width of said chassis, wherein said length extends along the longest dimension of said chassis;
one or more, preferably at least two, discrete elastically elongatable panels (11) each comprising an imaginary panel axis (PA) dividing said panel(s) (11) into two halves comprising an upper or back half and a lower or front half wherein the lower or front half is generally positioned closer to a front portion (F) of the article (1) compared to said upper or back half; and
one or more fastening members (12) joined to each of said one or more discrete elastically elongatable panels (11), and comprising an imaginary fastener axis (FA);
**characterized in that** the one or more fastening members (12) are joined to the upper or back half of said discrete elastically elongatable panels (11), and wherein the panel axis (PA) and the fastener axis (FA) are offset, and **in that** said panels (11) comprise at least one cover layer (13, 13') and an elastomeric film (14) attached to the cover layer (13, 13').

2. An absorbent article according to Claim 1 wherein the panel axis (PA) and the fastener axis (FA) are separated from each other by an axial distance (d_{A}), and preferably wherein the fastener axis (FA) substantially corresponds to a centerline of said fastening members (12).

3. An absorbent article according to any of the preceding Claims wherein the fastener axis (FA) of at least two, preferably each, oppositely disposed fastening members (12) substantially coincide such that the at least two, preferably each, oppositely disposed fastening members (12) share the same fastener axis, and preferably further crosses the longitudinal axis (y) when viewing the article in an open and laid flat position.

4. An absorbent article according to any of the preceding Claims wherein the panel axis (PA) and the fastener axis (FA) are offset such that the panel axis (PA) and the fastener axis (FA) are separated from each other by an axial distance (d_{A}) generally along an axis substantially parallel to the longitudinal axis (y) and substantially perpendicular to the panel axis (PA) and/or the fastener axis (FA); and/or wherein the panel(s) (11) comprise a longest length (Lmax_{P}) extending substantially parallel to the longitudinal axis (y) and generally positioned most adjacent to the chassis, wherein said longest length (Lmax_{P}) generally corresponds to a panel joint length of a panel joint region, the panel joint region being that portion of the panel(s) (11) that is joined to the chassis; and/or wherein the panel(s) (11) comprises a shortest length being located opposite the longest length (Lmax_{P}) and positioned furthest away from the chassis.

5. An absorbent article according to any of Claims 2 to 4 wherein the axial distance (d_{A}) is 0.03Lmax_{P} to 0.50Lmax_{P}, more preferably from 0.04 Lmaxp to 0.25Lmax_{P}, even more preferably from more than 0.04 Lmax_{P} to less than 0.20Lmaxₚ, even more preferably from 0.05 Lmax_{P} to 0.15Lmaxₚ.

6. An absorbent article according to any of the preceding Claims wherein the panel(s) (11) herein are positioned at a panel distance (D1) away from an article transversal centerline (x) towards the back (B) of the article, and wherein the ratio of the panel longest length and panel distance (Lmax_{P}/ D1) is from 0.30 to 1.0, preferably from 0.35 to 0.9, even more preferably from 0.40 to 0.85, even more preferably from 0.45 to 0.8.

7. An absorbent article according to any of the preceding Claims wherein the panels (11) are generally positioned such that no portion thereof extends beyond a terminal transversal edge (TE) of the article and preferably wherein a second panel distance (D2), extending from the terminal transversal edge (TE) and the closest portion of the panel (11) thereto, is greater than zero.

8. An absorbent article according to any of the preceding Claims comprising an elastic waistband (EWB) positioned on the back portion (B) of the article (1), preferably the elastic waistband (EWB) being positioned between the absorbent core and a terminal transversal edge (TE) of the article and spaced therefrom along the longitudinal axis (y).

9. An absorbent article according to Claim 8 wherein the fastener axis (FA) crosses the elastic waistband (EWB) and the longitudinal axis (y), preferably wherein the elastic waistband (EWB) is positioned to overlap the fastener axis (FA) and such that the longest length of the elastic waistband (EWB) is substantially parallel to an article transversal centerline (x).

10. An absorbent article according to any of the preceding Claims wherein the at least one cover layer (13, 13') and elastomeric film (14) are joined by mechanical bonding at a plurality of discrete bonding elements (15), and wherein each said panel (11) comprises a plurality of wrinkles having peaks (16) and troughs (17) formed on at least one of said cover layer (13, 13'), and preferably wherein the said each panel (11) comprises a plurality of openings (O) extending through the film (14) and/or the cover layer(s) (13, 13') and wherein said openings (O) are positioned outboard and/or inboard of said discrete bonding elements (15) so that said openings (O) and said bonding elements (15) do not substantially coincide.

11. An absorbent article according to any of the preceding Claims wherein the panel(s) (11) is breathable and has a water vapor transmission rate (WVTR) of less than about 2300 g/m²×24hr at an elongation of about 0% and a water vapor transmission rate (WVTR) of more than about 2800 g/m²×24hr at an elongation of about 100%, as measured according to the test method herein.

12. An absorbent article (1) according to any of the preceding Claims wherein the elastomeric film (14) comprises two surfaces and a skin on at least one of the surfaces.

13. An absorbent article (1) according to any of the preceding Claims comprising at least two, oppositely disposed elastically elongatable panels (11) joined to the chassis, with at least one said panels on either side of the back portion (B) of the chassis with the longitudinal axis (y) extending therebetween, and wherein said panels (11) are symmetric about at least one axis being parallel to the longitudinal axis (y) or the transversal axis (x).

14. An absorbent article (1) according to any of the preceding Claims wherein the at least one elastically elongatable panel (11) is joined to the chassis by mechanical bonding and/or adhesive at a first attachment zone of said panel, and preferably wherein said panel (11) is joined, preferably by mechanical bonding and/or adhesive, to a fastening member (12) at a second attachment zone of said panel oppositely disposed from said first attachment zone at an opposite end of said panel, and preferably wherein said absorbent article (1) comprises at least two of said panels (11) extending outboard of the chassis and oppositely disposed about the longitudinal axis (y) such that a centerline of said at least two of said panels (11) substantially correspond.

15. An absorbent article (1) according to any of the preceding Claims wherein the, preferably each, at least one cover layer (13, 13') is a nonwoven preferably having a basis weight of no more than 55 g/m² and preferably more than 17 g/m², preferably from 18 g/m² to 40 g/m², even more preferably from greater than 22 g/m² to less than 35 g/m², even more preferably from 23 g/m² to 30 g/m²; and/or wherein the elastically elongatable panel(s) (11) comprise at least two cover layers (13, 13') positioned on opposite faces of the film (14) such that said film (14) is sandwiched between said cover layers and wherein the basis weight of said cover layers is the same or different, and when different is different by a factor of at least 1.2, preferably at least 1.4, even more preferably from 1.5 to 3.
